# EUROPEAN PATENT APPLICATION

(11) **EP 2 065 062 A1**
(43) Date of publication of application: **03.06.2009**
(21) Application number: 08166474.0
(22) Date of filing: 13.10.2008
(51) Int. Cl.: A61M 5/145, H01L 41/02

(54) **Syringe pump**

(30) Priority: 15.10.2007 KR 20070103742
(71) Applicant: KOREA INSTITUTE OF MACHINERY & MATERIALS, Daejeon 305-343 (KR)
(72) Inventor: Ham, Young-Bog, Yuseong-gu 305-509, DAEJEON (KR); Park, Jung-Ho, Yuseong-gu 305-720, Daejeon (KR); Yun, So-Nam, 305-721, DAEJEON (KR); Seo, Woo-Suk, 305-804, DAEJEON (KR); Chung, Tae-Young, Yuseong-gu 305-500, DAEJEON (KR)
(74) Representative: Jacobson, Claude

(57) **Abstract**

A syringe pump comprising a linear actuator applying a linear motion of a piezoelectric linear motor (3) in moving the piston (2), such that suction and exhaustion of the fluid can be performed more precisely through controlling of power supply with respect to the piezoelectric linear motor.
The syringe pump includes a cylinder (1) which includes a receiving space, a piston (3) which is mounted in the cylinder to pump liquid or powder in and out of the cylinder, and a piezoelectric linear actuator (3) which moves the piston in a reciprocating manner.

## Description

### BACKGROUND OF THE INVENTION

### Field of the Invention

The present invention relates to a syringe pump for suction and exhaustion of a fixed quantity, and more particularly to a syringe pump, which is capable of taking in and out a fixed quantity of liquid or powder (hereinafter, collectively referred to as "fluid") more precisely by moving a piston using a linear actuator.

Especially, the above syringe pump uses the linear actuator which applies linear motion of a piezoelectric motor in order to move the piston, such that suction and exhaustion of the fluid can be performed more precisely through controlling of power supply with respect to the piezoelectric linear motor.

### Description of the Related Art

Generally, a fixed quantity feeder system is used to supply a precise quantity of chemical solution in a laboratory or medical substance as a medical instrument.

In such a fixed quantity feeder system, a stepping motor is generally used as a driving means for controlling the movement of a piston.

However, the conventional fixed quantity feeder system has such a complicated structure that manufacturing thereof is difficult. Furthermore, since the structure is hard to be in a compact size, handling of the system is inconvenient.

In addition, the stepping motor conventionally applied in the fixed quantity feeder system may sometimes cause vibration or resonance at certain frequency, thereby deteriorating the function of supplying a fixed quantity fluid.

### SUMMARY OF THE INVENTION

Therefore, the present invention has been made in view of the above problems, and it is an object of the present invention to provide a syringe pump, capable of controlling movement of a piston more precisely.

Especially, it is another object of the present invention to provide a syringe pump, capable of controlling a moving distance of a piston more precisely by intermittently supplying power to a linear actuator that moves the piston, and accordingly precisely controlling quantity of fluid being taken in and out of a cylinder.

More preferably, it is yet another object of the present invention to provide a syringe pump, capable of reducing the whole size of the pump by using the piezoelectric linear motor as a linear actuator.

In accordance with an aspect of the present invention, there is provided a syringe pump comprising a cylinder which includes a receiving space, a piston which is mounted in the cylinder to pump liquid or powder in and out of the cylinder, and a piezoelectric linear actuator which moves the piston in a reciprocating manner.

In addition, the piezoelectric linear actuator may comprise a piezoelectric motor constituted by piezoelectric mediums.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a sectional view of a syringe pump according to an embodiment of the present invention;
FIG. 2 is an exploded perspective view showing a piston and an actuator of the syringe pump shown in FIG. 1;
FIG. 3 is a sectional view of a syringe pump according to another embodiment of the present invention; and
Fig. 4 is an exploded perspective view showing a piston and an actuator of the syringe pump shown in FIG. 3.

### DETAILED DESCRIPTION OF THE PREFERRED EMBODIMENTS

Hereinafter, an exemplary embodiment of the present invention will be described in detail with reference to the accompanying drawings such that those who are skilled in the art can easily understand and implement the present invention.

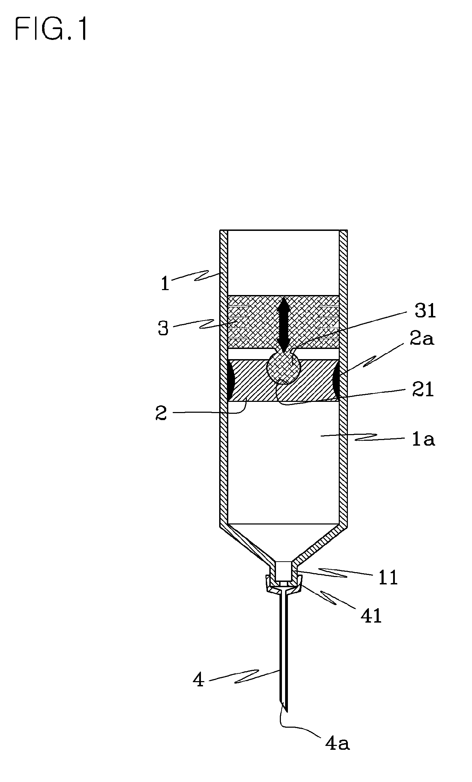

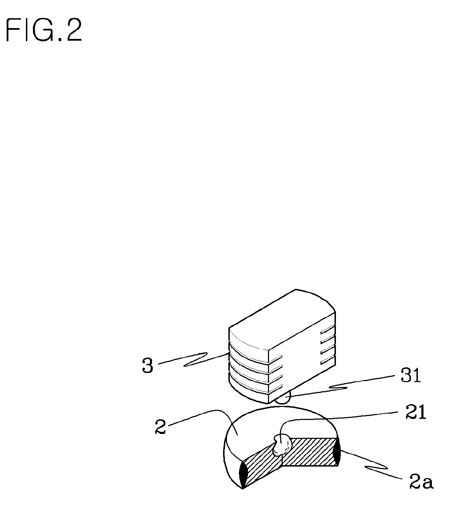

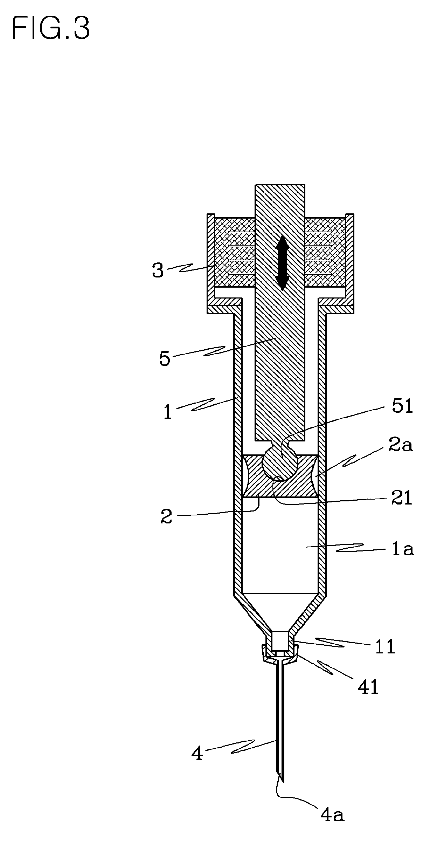

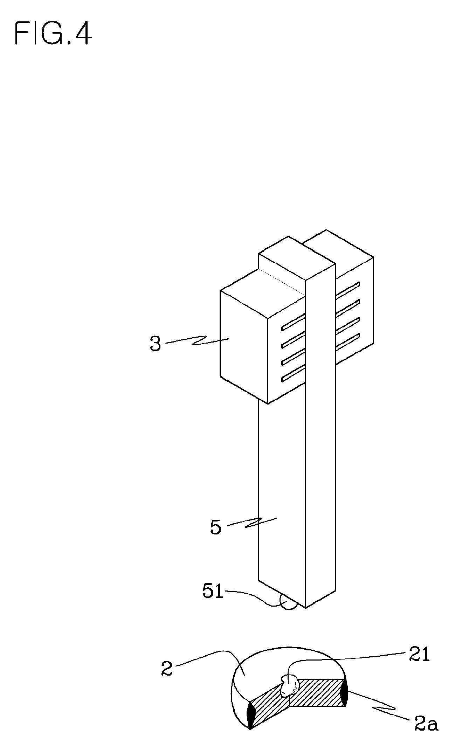

FIG. 1 is a sectional view of a syringe pump according to an embodiment of the present invention, and FIG. 2 is an exploded perspective view showing a piston and an actuator of the syringe pump shown in FIG. 1. FIG. 3 is a sectional view of a syringe pump according to another embodiment of the present invention, and Fig. 4 is an exploded perspective view showing a piston and an actuator of the syringe pump shown in FIG. 3.

As shown in the drawings, the syringe pump includes a linear actuator 3.

More specifically, the syringe pump according to the embodiment of the present invention comprises a cylinder 1 which includes a receiving space, a piston 2 which is mounted in the cylinder 1 to move liquid or powder in and out with respect to the cylinder 1, and a linear motor 3 which moves the piston 2 in a reciprocating motion.

In the above-structured syringe pump with the piezoelectric linear motor, the cylinder 1 and the piston 2 are used to take in certain liquid or micro powder and exhaust the liquid or powder to a desired place, having a similar structure with a syringe. The liquid and powder will be referred to as fluid.

That is, the cylinder 1 is formed as a tube holding a receiving space 1a for receiving the fluid. As shown in FIG. 1 and FIG. 3, one end of the cylinder 1 is totally open while the other end is sealed but having an inlet and outlet hole to let the fluid move in and out.

The cylinder 1 further includes a needle connection part 11 having a small diameter at the inlet and outlet hole such that the fluid can be supplied to or taken in from a narrow space as desired. Additionally, a needle 4 is removably connected to the needle connection part 11.

As shown in FIGs. 1 and 3, the needle 4 comprises a longitudinal fluid hole 4a having a very small diameter, and a piston connection part 41 fixed to the needle connection part 11.

The above cylinder 1 and needle 4 are structured in substantially the same manner as those of a general syringe. Therefore, the structure of the present invention can be achieved by adding a piston which includes the linear actuator 3 to the general conventional syringe.

The linear actuator 3 constituting the syringe pump, as shown in the drawings, is preferably implemented by a piezoelectric linear motor in order to reciprocate the piston 2.

The piezoelectric linear motor is a sort of motor that applies the principle in that a piezoelectric element contracts and expands according to power supply. As shown in the drawings, the piezoelectric linear motor includes a plurality of legs extending and contracting in a certain direction like a motion of an inchworm in accordance with power supply, thereby moving by itself or moving other parts in a pushing manner. Since the piezoelectric linear motor is generally known and applied in the art, detailed description thereof will be omitted herein.

In the above structure, as shown in FIG. 1 and FIG. 2, the piston 2 includes a connection recess 21 having a spherical shape for connection with a connection protrusion 31 having a spherical shape formed on a bottom surface of the linear actuator 3, so that the linear actuator 3 can be directly connected with the piston 2.

In a case where the piston 2 is thus directly connected to the linear actuator 3, the piston 2 is moved as the linear actuator 3 moves along an inner wall of the cylinder 1.

In other words, the linear actuator 3 shown in FIG. 1 and FIG. 2 can move the piston 2 by moving by itself.

Another method for achieving connection between the piston 2 and the linear actuator 3 is illustrated in FIG. 3 and FIG. 4.

As shown in the drawings, a rod 5 may be additionally mounted between the piston 2 and the linear actuator 3.

More specifically, as shown in FIG. 3 and FIG. 4, the rod 5 is used to connect the piston 2 to the linear actuator 3 and therefore, the piston 2 is moved as the rod 5 is moved up and down by the operation of the linear actuator 3. That is, the linear actuator 3 pushes the rod 5, while being fixed to the inner wall of the cylinder 1.

Furthermore, when the piston 2 is connected thus using the rod 5, the linear actuator 3 used in the embodiment of the present invention can also be easily adopted in existing syringe pumps.

As shown in the drawings, a connection protrusion 51 having a spherical shape is formed at an end of the rod 5, facing the piston 2 so as to connect the rod 5 with the piston 2. In addition, the piston 2 is formed with the connection recess 21 of a spherical shape for force-fit with the connection protrusion 51.

Thus, since connection parts of the rod 5 and the piston 2 are structured in the form of the spherical protrusion and the spherical recess, respectively, the piston 2 can be moved vertically in the cylinder 1 even when the rod 5 is inclined to a certain direction.

In addition, a sealant member 2a is mounted around the outer circumference of the piston 2 to prevent leakage of the fluid through a gap between the piston 2 and the cylinder 1.

In the above-structured syringe pump, the linear actuator 3 may have various modified configurations according to the present invention. Also, the embodiment is illustrated in the drawings only by way of example, but does not limit the present invention.

As can be appreciated from the above description, in accordance with a syringe pump according to the embodiment of the present invention, control of a moving distance of a piston can be performed more precisely by intermittently supplying power to a linear actuator. As a result, quantity of fluid taken in and out of a cylinder can be precisely controlled.

Especially, since the piezoelectric linear motor is used as a linear actuator for operating the piston, the syringe pump can be achieved in a compacter size.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A syringe pump comprising:
a cylinder which includes a receiving space;
a piston which is mounted in the cylinder to pump liquid or powder in and out of the cylinder; and
a piezoelectric linear actuator which moves the piston in a reciprocating manner.

2. The syringe pump according to claim 1, wherein the piezoelectric linear actuator comprises a piezoelectric linear motor performing a linear motion.

3. The syringe pump according to claim 1, further comprising a rod mounted between the piston and the linear actuator.

4. The syringe pump according to claim 2, further comprising a rod mounted between the piston and the linear actuator.

5. The syringe pump according to claim 3, wherein the rod includes a connection protrusion formed in a spherical shape on a surface thereof facing the piston, and the piston includes a connection recess formed in a spherical shape and ball-jointed with the connection protrusion.

6. The syringe pump according to claim 4, wherein the rod includes a connection protrusion formed in a spherical shape on a surface thereof facing the piston, and the piston includes a connection recess formed in a spherical shape and ball-jointed with the connection protrusion.

7. The syringe pump according to claim 5, further comprising a sealant member mounted around the outer circumference of the piston.

8. The syringe pump according to claim 6, further comprising a sealant member mounted around the outer circumference of the piston.

9. The syringe pump according to claim 7, further comprising:
a needle connection part having a small diameter, being disposed an inlet and outlet end; and
a needle removably connected to the needle connection part.

10. The syringe pump according to claim 8, further comprising:
a needle connection part having a small diameter, being disposed an inlet and outlet end; and
a needle removably connected to the needle connection part.
